# FASCICULE DE BREVET EUROPEEN

(11) **EP 0 832 293 B1**
(45) Date de publication et mention de la délivrance du brevet: **12.04.2000**
(21) Numéro de dépôt: 96920878.4
(22) Date de dépôt: 31.05.1996
(51) Int. Cl.: C12Q 1/68, C07H 21/04, C12P 19/34

(54) **PROCEDE DE MISE EN EVIDENCE DE MICROORGANISMES THERMORESISTANTS POUVANT CONTAMINER CERTAINES DENREES ALIMENTAIRES**
VERFAHREN ZUR BESTIMMUNG VON THERMORESISTENT-MIKROORGANISMEN MIT DER FAEHIGKEIT GEWISSE NAHRUNGSMITTEL ZU KONTAMINIEREN
METHOD FOR DETECTING HEAT-RESISTANT MICRO-ORGANISMS CAPABLE OF CONTAMINATING CERTAIN FOOD PRODUCTS

(30) Priorité: 02.06.1995 FR 9506578
(43) Date de publication de la demande: 01.04.1998
(73) Titulaire: Ultra Propre Nutrition Industrie Recherche (U.N.I.R.), 75008 Paris (FR)
(72) Inventeur: CHRZAVZEZ, Emmanuelle épouse TADDEI, F-67240 Bischwiller (FR); AUFRERE, Robert, F-92120 Montrouge (FR)
(74) Mandataire: Warcoin, Jacques
(86) Numéro de dépôt international: FR9600821
(87) Numéro de publication internationale: WO9638587

(56) Documents cités:
- LETTERS IN APPLIED MICROBIOLOGY, vol. 19, 24 Octobre 1994, pages 268-72, XP000609324 STUBBS, S. ET AL.: "Differentiation of the spoilage yeast Zygosaccharomyces bailli from other Zygosaccharomyces species using 18S rDNA as target for a non-radioactive ligase detection reaction"
- PLANT PATHOLOGY, vol. 42, no. 6, 1993, pages 873-81, XP000444029 POUPARD, P. ET AL.: "Molecular characterization of Pseudocercosporella herpotrichoides isolates by amplification of ribosomal DNA internal transcribed spacers"
- MYCOLOGIA, vol. 87, no. 2, Mars 1995, pages 210-22, XP000609217 BERBEE, M. ET AL.: "Is Penicillium monophyletic? An evolution of phylogeny in the family Trichocomaceae from its 18S, 5S and ITS ribosomal DNA sequence data"
- PHYSIOLOGICAL AND MOLECULAR PLANT PATHOLOGY, vol. 39, 1991, pages 1-11, XP000609318 NAZAR, R. ET AL: "Potential use of PCR-amplified ribosomal intergenic sequences in the detection and differentiation of verticillum wilt pathogens"
- WHITE, T. ET AL: "Amplification and direct sequencing of fungal ribosomal RNA genes for phylogenetics" 1990 , ACADEMIC PRESS INC , SAN DIEGO USA XP002017490 PCR PROTOCOLS: A guide to methods and applications. Innis, m. et al (ED); see p. 315-18, fig 2.

### LISTE DE SEQUENCES

(1) INFORMATIONS GENERALES:
   (i) DEPOSANT:
      (A) NOM: UNIR
      (B) RUE: 46 RUE DU BAC
      (C) VILLE: PARIS
      (E) PAYS: FRANCE
      (F) CODE POSTAL: 75007
   (iii) NOMBRE DE SEQUENCES: 12
(2) INFORMATIONS POUR LA SEQ ID NO: 1:
   (i) CARACTERISTIQUES DE LA SEQUENCE:
      (A) LONGUEUR: 186 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   CCGAGTGCGG GTCCCTCGTG CCCCAACCTC CAACCCGTGT TGACCGACAC CTGTTGCTTC 60
   GGCGGGCCCG CCCATGGGCT CCCGCCCGGC CGCCGGGGGG CCTCGTCGCC CCCGGGCCCG 120
   CCGCCCGCCG AAGACCCCTC GAACGCTGCC TTGAAGGTTG CCGTCTGAGT ATAAAATCAA 180
   TCGTTA 186
(2) INFORMATIONS POUR LA SEQ ID NO: 2:
   (i) CARACTERISTIQUES DE LA SEQUENCE:
      (A) LONGUEUR: 187 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   GCTAACCCTC CAGCCCGGCT GGTGTGTTGG GCCGCCGTCC CCTCCGGGGG ACGGGCCCGA 60
   AAGGCAGCGG CGGCGCCGTC CGGTCCTCGA GCGTATGGGG CTTTGTCACA CGCTCTGGTA 120
   GGCCCGGCCG GCTTGCTGGC AAACGACCTC ACGGTCACCT AACTTCTCTC TTAGGTTGAC 180
   CTCGGAT 187
(2) INFORMATIONS POUR LA SEQ ID NO: 3:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3:
   CCGAGTGAGG GCCTCTGGGT CCAACTTCCC ACCCGTGTCT ATCGTACCTT GTTGCTTCGG 60
   CGGGCCCGCC GTTTCGACGG CCGCCGGGGA GGCCTCGCGC CCCCGGGCCC GCGCCCGCCG 120
   AAGACCCCAA CATGAACGCT GTTCTGAAAG TATGCAGTCT GAGTTGATTA TCATAATCAG 180
   TTA 183
(2) INFORMATIONS POUR LA SEQ ID NO: 4:
   (i) CARACTERISTIQUES DE LA SEQUENCE:
      (A) LONGUEUR: 176 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   GCTGCCCTCA GGCACGGCTT GTGTGTTGGG CGTCCCCTCT CCCGGGAACG GGCCCGAAAG     60
   GCAGCGGCGG CACCGCGTCC GGTCCTCGAG CGTATGGGGC TTTGTCACCC GCTCTGTAGG    120
   CCCGGCCGGC GCCAGCCGAC ACCCAACTTT ATTTCTAAGG TTGACCTCGG ATCAGG        176
(2) INFORMATIONS POUR LA SEQ ID NO: 5:
   (i) CARACTERISTIQUES DE LA SEQUENCE:
      (A) LONGUEUR: 279 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   TAGAAAAATT TCTGATTGAC GAGTCTGGAG CAGTTGTGTT CTTTCTGTTT TTTTTAAGGC     60
   CTGCGCTTAA TTGCCGTCTA GAGCGGAGGG AGTTAAGCAT AGTTGCCTTT GGCTTTCAAT    120
   TTACACACAG TGGAGTTTCT ACTTTTTTTA TTCTTCTTTG GGAGGATGGG TTCGTCCCGC    180
   TCCCAGAGGT AAACACAAAC AATTTTTTTT ATTTTATTTT ATTTTATTAT TATAATAATA    240
   ATAATACAGT CAAAACGAAT ACTAAAAAAA AAATATTCA                           279
(2) INFORMATIONS POUR LA SEQ ID NO: 6:
   (i) CARACTERISTIQUES DE LA SEQUENCE:
      (A) LONGUEUR: 237 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   CCTTCTCAAA CATTCGTGTT TGGTAGTGAG TGATACTCTG TTTTATATTT GGGTTAACTT     60
   GAAATTGCAA GCCTTTTGGG ACGCGTGTGG GTGAGTTTTA GGCCGGAAAC GTCTTGCTCT    120
   CCTCTTTCCT AACCAAATGT CGTATTAGGT TTTACCGACT CCGACAGACG GGACTAGGAG    180
   ATTGGGTGAG TGATAGCAAT ATCGAGCTCT GCCTAATTTT TTTTTTTGCG CGCCTTG       237
(2) INFORMATIONS POUR LA SEQ ID NO: 7:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 7:
   GGTTGCCGTC TGAGTATAAA ATCAA 25
(2) INFORMATIONS POUR LA SEQ ID NO: 8:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 8:
   GAAGTTAGGT GACCGTGAGG TCGTT 25
(2) INFORMATIONS POUR LA SEQ ID NO: 9:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 9:
   AAGACCCCAA CATGAACGCT GTTCT 25
(2) INFORMATIONS POUR LA SEQ ID NO: 10:
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 10:
   TCCGAGGTCA ACCTTAGAAA TAAAGTT                                    27
(2) INFORMATIONS POUR LA SEQ ID NO: 11:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 11:
   TAGAGCGGAG GGAGTTAAGC ATAGT                                      25
(2) INFORMATIONS POUR LA SEQ ID NO: 12:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 12:
   TGGTTAGGAA AGAGGAGAGC AAGAC                                      25

## Description

La présente invention concerne une méthode de détection rapide de microorganismes eucaryotes thermorésistants pouvant contaminer certains produits dans le domaine agro-alimentaire, notamment des produits à base de fruits.

Plus particulièrement, les microorganismes étudiés sont deux champignons filamenteux, *Byssochlamys nivea* et *Neosartorya fischeri* et une levure, *Zygosaccharomyces bailii.*

Ces contaminants de produits alimentaires peuvent se développer sur des produits à base de fruits, leur communiquant, notamment pour *Byssochlamys nivea*, des notes désagréables de type plastique ou antiseptique, la croissance de *Neosartorya fischeri* est responsable d'une altération et de l'apparition de mycoroxines, alors que celle de *Zygosaccharomyces bailii* provoque une formation de gaz.

Ces deux champignons filamenteux, et en particulier leurs spores, présentent une themorésistance très élevée qui leur permet de résister aux barèmes de pasteurisation utilisés couramment dans les industries agro-alimentaires.

Les tests permettant la mise en évidence de ces microorganismes existant à l'heure actuelle consistent à cultiver les échantillons sur un milieu sélectif en mettant à profit, comme élément de sélection, la thermorésistance des champignons filamenteux ou la résistance de la levure à certains agents conservateurs utilisés classiquement dans le domaine tels que l'acide benzoïque. Toutefois, ce type de détection implique des cultures et, qui plus est, des cultures assez longues, à savoir de plusieurs jours à plusieurs semaines, ce qui les rend pratiquement inutilisables dans le domaine agro-alimentaire puisque cela impliquerait de conserver les produits éventuellement contaminés pendant des temps très longs.

On est donc à la recherche d'un test de détection permettant la mise en évidence très rapide de ces microorganismes, ledit test pouvant être effectué en quelques heures au plus, afin de savoir rapidement si le produit échantilloné peut ou non continuer à être traité dans les étapes ultérieures.

C'est pourquoi, la présente invention propose un procédé permettant la mise en évidence de *Byssochlamys nivea, Neosartorya fischeri* et *Zygosaccharomyces bailii* par amplification de l'ADN génomique des microorganismes ciblés en utilisant comme amorces un couple de séquences comprises dans les espaceurs transcrits internes (ITS) de l'unité ribosomique :
- pour *Byssochlamys nivea,*
   - sur ITS1 correspondant à SEQ ID 1,
   - sur ITS2 correspondant à SEQ ID 2,
- pour *Neosartorya fischeri,*
   - sur ITS1 correspondant à SEQ ID 3,
   - sur ITS2 correspondant à SEQ ID 4,
- pour *Zygosaccharomyces bailii*
   - sur ITS1 correspondant à SEQ ID 5
   - sur ITS2 correspondant à SEQ ID 6.

Dans les séquences ITS1 et ITS2 on choisira plus particulièrement,
- pour *Byssochlamys nivea* la séquence SEQ ID 7 et ID 8,
- pour *Neosartorya fischeri* SEQ ID 9 et ID 10,
- pour *Zygosaccharomyces bailii* SEQ ID 11 et ID 12.

Parmi les méthodes d'amplification utilisables, on utilisera plus particulièrement la méthode dite "PCR" ("Polymerase Chain Reaction").

Les essais, rapportés notamment dans les exemples, montrent que ces amorces sont parfaitement discriminantes et permettent de distinguer les microorganismes recherchés et les microorganismes, même très voisins, et apparentés.

La présente invention concerne également un procédé de mise en évidence de ces microorganismes dans des produits agro-alimentaires, notamment des produits agro-alimentaires à base de fruits et plus particulièrement des produits alimentaires à base de fraises, dans lequel on prétraite l'échantillons contenant les fruits afin de libérer et concentrer les spores ou les cellules, de diminuer ou de supprimer l'action des inhibiteurs de la Taq Polymérase et d'extraire l'ADN des spores ou des cellules.

Plus particulièrement, les échantillons contenant des produits solides seront traités par un mélange de cellulase/hémicellulase afin de les liquéfier puis filtrés et centrifugés dans des conditions permettant d'obtenir un produit contenant les cellules dont on pourra extraire l'ADN.

Les échantillons contenant la plupart du temps des inhibiteurs de la Taq Polymérase, il est nécessaire de prévoir une étape permettant de diminuer l'activité des inhibiteurs, soit par dilution, soit par traitement de l'échantillon au phénol-chloroforme et précipitation à l'alcool.

Bien que la présente invention soit plus particulièrement destinée à l'industrie agro-alimentaire, il est évident qu'elle pourra également être utilisée afin de mettre en évidence les microorganismes en cause dans d'autres échantillons qui pourraient n'avoir aucun rapport avec l'industrie agro-alimentaire.

Enfin, la présente invention concerne des amorces telles que définies précédemment ainsi que des trousses de détection mettant en oeuvre lesdites amorces.

D'autres caractéristiques et avantages de la présente invention apparaitront à la lecture des exemples ci-après.

### EXEMPLE 1 - PROCEDE D'EXTRACTION D'ADN ET D'AMPLIFICATION

I) A partir d'environ 5 mm² de mycelium de champignon, 10⁵ spores de champignon ou 10⁶ cellules de levure :
   - dans un microtube à vis (type Eppendorf 1,5 ml), mise en suspension des microorganismes dans 200 µl de tampon Tris 50 mM EDTA 20 mM SDS 0,8 %, pH 8,5, auxquels sont ajoutés 100 µl de billes de verre (diamètre 0,25 - 0,5 mm),
   - vortexer 1 minute à fréquence maximum,
   - incubation du mélange pendant 15 minutes à 100°C (eau bouillante),
      * pour le traitement des spores, on remplace le vortexage et l'incubation par :
         - congélation dans N₂ liquide, ébullition 100°C 5 minutes,
         - recongélation dans N₂ liquide, ébullition 100°C 10 minutes,
   - centrifugation 1 minute à 10 000 g (centrifugeuse de paillasse),
   - récupération du surnageant,
   - dilution du surnageant par 10 dans l'eau.

   Ce surnageant dilué est utilisé directement pour l'amplification.
II) L'amplification est faite dans un volume final de 25 µl contenant 20 mM Tris-HCl pH 8,5, (NH₄)₂SO₄ 16 mM, MgCl₂, 2,5 mM, 150 µg/ml de sérum albumine bovine, 0,2 µM de chaque dNTP et 100 µM de chacun des oligonucléotides amorce. Deux unités de Taq DNA polymérase (BioTaq, Bioprobe systems, France) sont utilisées par réaction. La réaction d'amplification est effectuée dans le thermocycler perkin Elmer 2400 (Perkin Elmer Corp., USA) de la façon suivante :
   - pour *Zygosaccharomyces, Byssochlamys* et *Neosartoria*
      . 15 secondes à 94°C
      . 10 secondes à 58°C
      . 20 secondes à 72°C

      Ce cycle est répété 30 fois. Il est suivi par une élongation terminale de 5 minutes à 72°C.
III) Les produits obtenus sont visualisés sous UV après migration sur gel d'électrophorèse de composition suivante : tampon TBEx1, Bromure d'éthidium 1 µg/ml, agarose 0,8 %.

### EXEMPLE 2

Ci-après on trouvera les résultats observés par mise en oeuvre du procédé selon la présente invention sur différentes souches proches des souches à détecter montrant l'excellente discrimination apportée par les jeux d'amorces revendiqués.

Les amorces utilisées sont :
- SEQ ID 7 et 8 pour *Byssochlamys nivea,*
- SEQ ID 9 et 10 pour *Neosartorya fischeri,*
- SEQ ID 11 et 12 pour *Zygosaccharomyces bailii*.

Il convient de noter que *A. fumigatus* répond à la sonde car il s'agit de la forme asexuée de N. *fischeri.*

| Oligonucléotides-amorce spécifiques de Neosartoria fischeri | | | | |
|---|---|---|---|---|
| Genre | Espèce | Souche | T° an. | Réponse PCR |
| Aspergillus | floriformis | M 93 2663 | 57°C | négative |
| Aspergillus | fumigatus | M 70 665 | 57°C | positive |
| Aspergillus | fumigatus | M 88 2521 | 57°C | positive |
| Byssochlamys | nivea | M 90 1496 | 57°C | négative |
| Byssochlamys | nivea | M 93 2932 | 57°C | négative |
| Emericella | nidulans | M 88 2519 | 57°C | négative |
| Emericella | nidulans var.acristata | M 84 2558 | 57°C | négative |
| Emericella | nidulans var.dentata | M 84 2556 | 57°C | négative |
| Emericella | nidulans var.lata | M 68 1987 | 57°C | négative |
| Emericella | nidulans var.shinulata | M 84 9557 | 57°C | négative |
| Eupenicillium | brefeldianum | M 89 2573 | 57°C | négative |
| Eurotium | amstelodami | M 50 142 | 57°C | négative |
| Eurotium | amstelodami | M 88 2536 | 57°C | négative |
| Eurotium | repens | M 66 2534 | 57°C | négative |
| Mariannae | elegans | M 95 3777 | 57°C | négative |
| Monascus | ruber | M 65 1079 | 57°C | négative |
| Neosartoria | fennelliae | M 93 2982 | 57°C | positive |
| Neosartoria | fennelliae | M 95 3781 | 57°C | positive |
| Neosartoria | fennelliae | M 953780 | 57°C | positive |
| Neosartoria | pseudofischeri | M 93 2986 | 57°C | positive |
| Neosartoria | pseudofischeri | M 95 3784 | 57°C | positive |
| Neosartorya | aurata | M 93 2980 | 57°C | positive |
| Neosartorya | aureola | M 90 3623 | 57°C | positive |
| Neosartorya | aureola | M 93 2981 | 57°C | positive |
| Neosartorya | aureola | M 95 3785 | 57°C | positive |
| Neosartorya | fennelliae | M 93 2983 | 57°C | positive |
| Neosartorya | fischeri | M 90 2660 | 57°C | positive |
| Neosartorya | fischeri var.fischeri | M 88 2482 | 57°C | positive |
| Neosartorya | fischeri var.fischeri | M 89 2707 | 57°C | positive |
| Neosartorya | fischeri var.fischeri | M 90 3618 | 57°C | positive |
| Neosartorya | fischeri var.fischeri | M 90 3619 | 57°C | positive |
| Neosartorya | fischeri var.fischeri | M 90 3620 | 57°C | positive |
| Neosartorya | fischeri var.fischeri | M 90 3621 | 57°C | positive |
| Neosartorya | fischeri var.glabra | M 87 3513 | 57°C | positive |
| Neosartorya | fischeri var.glabra | M 88 2480 | 57°C | positive |
| Neosartorya | fischeri var.glabra | M 88 3577 | 57°C | positive |
| Neosartorya | fischeri var.glabra | M 90 3622 | 57°C | positive |
| Neosartorya | fischeri var.glabra | M 92 2891 | 57°C | positive |
| Neosartorya | fischeri var.glabra | M 92 2925 | 57°C | positive |
| Neosartorya | fischeri var.spinosa | M 88 2480 | 57°C | positive |
| Neosartorya | fischeri var.spinosa | M 88 3574 | 57°C | positive |
| Neosartorya | fischeri var.spinosa | M 90 2749 | 57°C | positive |
| Neosartorya | fischeri var.spinosa | M 92 2862 | 57°C | positive |
| Neosartorya | fischeri var.spinosa | M 92 2910 | 57°C | positive |
| Neosartorya | fischeri var.spinosa | M 92 2924 | 57°C | positive |
| Neosartorya | fischeri var.spinosa | M 93 2935 | 57°C | positive |
| Neosartorya | hiratsukae | M 95 3782 | 57°C | positive |
| Neosartorya | quadricincta | M 93 2984 | 57°C | positive |
| Neosartorya | quadricincta | M 95 3786 | 57°C | positive |
| Neosartorya | spathulata | M 93 2978 | 57°C | positive |
| Neosartorya | spathulata | M 93 2979 | 57°C | positive |
| Neosartorya | stramenia | M 93 2985 | 57°C | positive |
| Neosartorya | tatenoï | M 95 3783 | 57°C | positive |
| Paecilomyces | carneus | M 52 907 | 57°C | négative |
| Paecilomyces | carneus | M 95 3776 | 57°C | négative |
| Paecilomyces | elegans | M 79 1670 | 57°C | négative |
| Paecilomyces | farinosus | M 75 3028 | 57°C | négative |
| Paecilomyces | fumoso-roseus | M 52 597 | 57°C | négative |
| Paecilomyces | inflatus | M 57 1387 | 57°C | négative |
| Paecilomyces | javanicus | M 69 3302 | 57°C | négative |
| Paecilomyces | javanicus | M 90 2684 | 57°C | négative |
| Paecilomyces | lilacinus | M 2689 | 57°C | négative |
| Paecilomyces | lilacinus | M 70 3099 | 57°C | négative |
| Paecilomyces | marquandii | M 50 85 | 57°C | négative |
| Paecilomyces | niphetodes | M 79 3238 | 57°C | négative |
| Paecilomyces | niphetodes | M 95 3778 | 57°C | négative |
| Paecilomyces | variotii | M 93 3701 | 57°C | négative |
| Paecilomyces | zolerniae | M 93 3648 | 57°C | négative |
| Penicillium | chrysogenum | M 47 673 | 57°C | négative |
| Penicillium | islandicum | M 73 2233 | 57°C | négative |
| Penicillium | islandicum | M 73 2233 | 57°C | négative |
| Penicillium | purpurogenum | M 90 2600 | 57°C | négative |
| Penicillium | variabile | M 78 3162 | 57°C | négative |
| Penicillium | viridicatum | M 88 2485 | 57°C | négative |
| Talaromyces | bacilliformis | M 88 1497 | 57°C | négative |
| Talaromyces | bacilliformis | M 88 2511 | 57°C | négative |
| Talaromyces | bacilliformis | M 90 1493 | 57°C | négative |
| Talaromyces | bacilliformis | M 90 2646 | 57°C | négative |
| Talaromyces | bacillisporus | M 88 2511 | 57°C | négative |
| Talaromyces | flavus | M 83 1179 | 57°C | négative |
| Talaromyces | flavus | M 88 2481 | 57°C | négative |
| Talaromyces | flavus | M 89 1489 | 57°C | négative |
| Talaromyces | flavus | M 93 2944 | 57°C | négative |
| Talaromyces | helicus var. helicus | M 88 2510 | 57°C | négative |
| Talaromyces | luteus | M 61 1858 | 57°C | négative |
| Talaromyces | sp. | M 92 2885 | 57°C | négative |
| Talaromyces | sp. | M 92 2887 | 57°C | négative |
| Talaromyces | sp. | M 92 2888 | 57°C | négative |
| Thermoascus | crustaceus | M 88 2540 | 57°C | négative |

Les souches proviennent du Museum National d'Histoire Naturelle de Paris

| Oligonucléotides-amorce spécifiques de Zygosaccharomyces bailii | | | | |
|---|---|---|---|---|
| Genre | Espèce | Souche | T° an. | Réponse PCR |
| Candida | inconspicua | M E10 | 50°C | négative |
| Candida | magnoliae | M E14 | 50°C | négative |
| Candida | mogii | M 95 3795 | 55°C | négative |
| Clavispora | lusitaniae | M E9 | 50°C | négative |
| Clavispora | lusitaniae | SIAS | 58°C | négative |
| Cytofilobasidium | | M E13 | 50°C | négative |
| Debaryomyces | hansenii | SIAS | 58°C | négative |
| Debaryomyces | hansenii | M E18 | 50°C | négative |
| Hyphopichia | | M E6 | 50°C | négative |
| Kluyveromyces | marxianus | M 95 3788 | 55°C | négative |
| | var.lactis | | | |
| Kluyveromyces | marxianus | M 95 3789 | 55°C | négative |
| | var.lactis | | | |
| Kluyveromyces | marxianus | M 95 3790 | 55°C | négative |
| | var.marxianus | | | |
| Kluyveromyces | marxianus | M 95 3791 | 55°C | négative |
| | var.marxianus | | | |
| Kluyveromyces | var.lactis | P 8 | 57°C | négative |
| Moniliella | | M E2 | 50°C | négative |
| Pichia | anomala | M E12 | 50°C | négative |
| Pichia | anomala | SIAS | 58°C | négative |
| Pichia | fermentans | SIAS | 58°C | négative |
| Pichia | guilliermondii | SIAS | 58°C | négative |
| Pichia | guilliermondii | M E17 | 50°C | négative |
| Pichia | pastoris | M 95 3792 | 55°C | négative |
| Pichia | pastoris | M 95 3793 | 55°C | négative |
| Rhodoturola | mucilaginosa | M E15 | 50°C | négative |
| Saccharomyces | bayanus | M 95 3796 | 55°C | négative |
| Saccharomyces | bayanus | P 562 | 57°C | négative |
| Saccharomyces | bayanus | P 563 | 57°C | négative |
| Saccharomyces | capsularis | M 95 3807 | 55°C | négative |
| Saccharomyces | cerevisiae | H 5035 | 57°C | négative |
| Saccharomyces | cerevisiae | H 5090 | 57°C | négative |
| Saccharomyces | cerevisiae | H 5130 | 57°C | négative |
| Saccharomyces | cerevisiae | H 5160 | 57°C | négative |
| Saccharomyces | cerevisiae | H 5200 | 57°C | négative |
| Saccharomyces | cerevisiae | H 5334 | 57°C | négative |
| Saccharomyces | cerevisiae | M E16 | 50°C | négative |
| Saccharomyces | cerevisiae | P 1 | 57°C | négative |
| Saccharomyces | cerevisiae | P 575 | 57°C | négative |
| Saccharomyces | cerevisiae | SIAS | 58°C | négative |
| Saccharomyces | exiguus | M 95 3797 | 55°C | négative |
| Saccharomyces | kluyveri | M 95 3798 | 55°C | négative |
| Saccharomyces | ludwigii | M 95 3806 | 55°C | négative |
| Saccharomyces | servazii | M 95 3799 | 55°C | négative |
| Saccharomyces | stellatus | M 95 3803 | 55°C | négative |
| Saccharomyces | stellatus | M 95 3804 | 55°C | négative |
| Saccharomyces | unisporus | M 95 3805 | 55°C | négative |
| Schyzosaccharomyces | pombe | H 5096 | 57°C | négative |
| Souche osmophile | | H 11 077 | 57°C | négative |
| Souche osmophile | | H 37 080 | 57°C | négative |
| Torulaspora | delbruckii | M 95 3794 | 55°C | négative |
| Torulaspora | delbruckii | P L121 | 57°C | négative |
| Torulaspora | delbruckii | SIAS | 58°C | négative |
| Trichosporum | capitatum | SIAS | 50°C | négative |
| Zygosaccharomyces | bailii | M 90 2638 | 58°C | positive |
| Zygosaccharomyces | bailii | M 90 2639 | 55°C | positive |
| Zygosaccharomyces | bailii | M 92 2895 | 55°C | positive |
| Zygosaccharomyces | bailii | M 92 2896 | 55°C | positive |
| Zygosaccharomyces | bailii | M 92 2897 | 58°C | positive |
| Zygosaccharomyces | bailii | M 92 2899 | 58°C | positive |
| Zygosaccharomyces | bailii | M 92 2900 | 58°C | positive |
| Zygosaccharomyces | bailii | M 92 2901 | 55°C | positive |
| Zygosaccharomyces | bailii | M 92 2902 | 55°C | positive |
| Zygosaccharomyces | bailii | M 92 2903 | 58°C | positive |
| Zygosaccharomyces | bailii | M 92 2904 | 55°C | positive |
| Zygosaccharomyces | bailii | M 92 2905 | 55°C | positive |
| Zygosaccharomyces | bailii | M 92 2906 | 55'C | positive |
| Zygosaccharomyces | bailli | M 92 2907 | 55°C | positive |
| Zygosaccharomyces | bailii | M 92 2908 | 58°C | positive |
| Zygosaccharomyces | bailii | M 92 2909 | 58°C | positive |
| Zygosaccharomyces | bailii | M 92 2911 | 55°C | positive |
| Zygosaccharomyces | bailii | M 92 2912 | 58°C | positive |
| Zygosaccharomyces | bailii | M 92 2913 | 58°C | positive |
| Zygosaccharomyces | bailii | M 92 2914 | 55°C | positive |
| Zygosaccharomyces | bailii | M 92 2915 | 58°C | positive |
| Zygosaccharomyces | bailii | M 92 2916 | 55°C | positive |
| Zygosaccharomyces | bailii | M 92 2917 | 58°C | positive |
| Zygosaccharomyces | bailii | M 92 2918 | 58°C | positive |
| Zygosaccharomyces | bailii | M 92 2920 | 55°C | positive |
| Zygosaccharomyces | bailii | M 92 2921 | 55°C | positive |
| Zygosaccharomyces | bailii | M 92 2922 | 58°C | positive |
| Zygosaccharomyces | bailii | M 92 2923 | 58°C | positive |
| Zygosaccharomyces | bailii | M E11 | 55°C | positive |
| Zygosaccharomyces | bailii | P L443 | 58°C | positive |
| Zygosaccharomyces | bisporus | DBVPG 6382 | 58°C | négative |
| Zygosaccharomyces | cidri | DBVPG 6385 | 55°C | négative |
| Zygosaccharomyces | fermentati | DBVPG 6297 | 55°C | négative |
| Zygosaccharomyces | florentinus | DBVPG 6186 | 55°C | négative |
| Zygosaccharomyces | microellipsoides | DBVPG 6188 | 55°C | négative |
| Zygosaccharomyces | mrakii | DBVPG 6289 | 55°C | négative |
| Zygosaccharomyces | rouxii | MUCL 30008 | 55°C | négative |
| Zygosaccharomyces | rouxii | SIAS | 58°C | négative |

### PROVENANCE DES SOUCHES

DBVPG: Dipartemento di Biologia Vegetale Perugia (Italie)
H: Société Heudebert
M: Museum National d'Histoire Naturelle de Paris
MUCL: Mycothèque de l'Université Catholique de Louvains
P: Société Pernod-Ricard
SIAS: Société SIAS france

## Revendications

1. Procédé permettant la mise en évidence de *Byssochlamys nivea, Neosartorya fischeri* et *Zygosaccharomyces bailii* par amplification de l'ADN génomique des microorganismes ciblés en utilisant comme amorce une séquence comprise dans les espaceurs transcrits internes (ITS) de l'unité ribosomique :
- pour *Byssochlamys nivea,*
- sur ITS1 correspondant à SEQ ID 1,
- sur ITS2 correspondant à SEQ ID 2,
- pour *Neosartorya fischeri,*
- sur ITS1 correspondant à SEQ ID 3,
- sur ITS2 correspondant à SEQ ID 4,
- pour *Zygosaccharomyces bailii*
- sur ITS1 correspondant à SEQ ID 5
- sur ITS2 correspondant à SEQ ID 6.

2. Procédé selon la revendication 1, caractérisé en ce que dans les séquences ITS1 et ITS2 on choisit plus particulièrement,
- pour *Byssochlamys nivea* les séquences SEQ ID 7 et 8,
- pour *Neosartorya fischeri* les séquences SEQ ID 9 et 10,
- pour *Zygosaccharomyces bailii* les séquences SEQ ID 11 et 12.

3. Procédé selon l'une des revendications 1 ou 2, caractérisé en ce que l'amplification est réalisée par la méthode PCR.

4. Procédé selon l'une des revendications 1 à 3 pour la mise en évidence de ces microorganismes dans des produits agro-alimentaires, notamment des produits agro-alimentaires à base de fruits et plus particulièrement des produits alimentaires à base de fraises, dans lequel on prétraite l'échantillon contenant les fruits afin de libérer et concentrer les spores ou les cellules, de diminuer ou de supprimer l'action des inhibiteurs de la Taq Polymérase et d'extraire l'ADN des spores ou des cellules.

5. Procédé selon la revendication 4, caractérisé en ce que les échantillons contenant les fruits sont traités par un mélange de cellulase/hémicellulase afin de les liquéfier, puis filtrés et centrifugés dans des conditions permettant d'obtenir un produit contenant les cellules dont on pourra extraire l'ADN.

6. Amorce ayant une séquence choisie dans les séquences SEQ ID 1 à 12.

7. Trousse de détection par amplification d'ADN et destinée à la mise en oeuvre du procédé selon l'une des revendications 3 à 5, caractérisée en ce qu'elle comporte des amorces selon la revendication 6.

## Patentansprüche

1. Verfahren, das den Nachweis von *Byssochlamys nivea, Neosartorya fischeri* und *Zygosaccharomyces bailii* durch Amplifikation von genomischer DNA der Ziel-Organismen gestattet, indem man als Ausgangsmaterial eine Sequenz verwendet, die in den inneren transskribierten Zwischenstücken (ITS) der ribosomischen Einheit enthalten ist:
- bei *Byssochlamys nivea*
- auf ITS1, entsprechend der SEQ ID 1,
- auf ITS2, entsprechend der SEQ ID 2,
- bei *Neosartorya fischeri*
- auf ITS1, entsprechend der SEQ ID 3,
- auf ITS2, entsprechend der SEQ ID 4,
- bei *Zygosaccharomyces bailii*
- auf ITS1, entsprechend der SEQ ID 5,
- auf ITS2, entsprechend der SEQ ID 6.

2. Verfahren nach Anspruch 1, dadurch gekennzeichnet, daß man in den Sequenzen ITS1 und ITS2 spezieller auswählt:
- bei *Byssochlamys nivea* die Sequenzen SEQ ID 7 und ID 8,
- bei *Neosartorya fischeri* die Sequenzen SEQ ID 9 und ID 10,
- bei *Zygosaccharomyces bailii* die Sequenzen SEQ ID 11 und ID 12.

3. Verfahren nach einem der Ansprüche 1 oder 2, dadurch gekennzeichnet, daß die Amplifikation durch das PCR-Verfahren durchgeführt wird.

4. Verfahren nach einem der Ansprüche 1 bis 3 für den Nachweis dieser Mikroorganismen in Nahrungsmittelprodukten, insbesondere Nahrungsmittelprodukten auf der Grundlage von Früchten und spezieller Nahrungsmittelprodukten auf der Grundlage von Erdbeeren, in dem man eine Probe, welche die Früchte enthält, vorbehandelt, um die Sporen oder die Zellen freizusetzen und zu konzentrieren, die Wirkung der Inhibitoren der Taq-Polymerase zu verringern oder zu supprimieren und die DNA der Sporen oder der Zellen zu extrahieren.

5. Verfahren nach Anspruch 4, dadurch gekennzeichnet, daß die Proben, welche die Früchte enthalten, mit einer Mischung aus Cellulase/Hemicellulase behandelt werden, um sie zu verflüssigen, dann unter Bedingungen filtriert und zentrifugiert werden, welche gestatten, daß man ein Produkt erhält, das die Zellen enthält, wovon man die DNA extrahieren kann.

6. Ausgangsmaterial, das eine Sequenz aufweist, die aus den Sequenzen SEQ ID 1 bis 12 ausgewählt ist.

7. Reagenziensatz zum Nachweis von DNA durch Amplifikation und zur Durchführung des Verfahrens nach einem der Ansprüche 3 bis 5 bestimmt, dadurch gekennzeichnet, daß er die Ausgangsmaterialien gemäß Anspruch 6 umfaßt.

## Claims

1. Process allowing the detection of *Byssochlamys nivea, Neosartorya fischeri* and *Zygosaccharomyces bailii* by amplification of the genomic DNA of the targeted microorganisms using, as primers, a sequence contained in the internal transcribed spacers (ITS) of the ribosomal unit:
- for *Byssochlamys nivea,*
- on ITS1 corresponding to SEQ ID 1,
- on ITS2 corresponding to SEQ ID 2,
for *Neosartorya fischeri,*
- on ITS1 corresponding to SEQ ID 3,
- on ITS2 corresponding to SEQ ID 4,
for *Zygosaccharomyces bailii*
- on ITS1 corresponding to SEQ ID 5,
- on ITS2 corresponding to SEQ ID 6.

2. Process according to Claim 1, characterized in that in the ITS1 and ITS2 sequences, there is chosen more particularly,
- for *Byssochlamys nivea,* the sequences SEQ ID 7 and 8,
- for *Neosartorya fischeri,* the sequences SEQ ID 9 and 10,
- for *Zygosaccharomyces bailii,* the sequences SEQ ID 11 and 12.

3. Process according to one of Claims 1 or 2, characterized in that the amplification is carried out by the PCR method.

4. Process according to one of Claims 1 to 3, for detecting these microorganisms in food products, particularly with fruit-based food products and more particularly strawberry-based food products, in which the sample containing the fruits is pretreated so as to liberate and concentrate the spores or the cells, to reduce or suppress the action of the inhibitors of Taq Polymerase and to extract the DNA from the spores or cells.

5. Process according to Claim 4, characterized in that the samples containing fruits are treated with a mixture of cellulase/hemicellulase so as to liquefy them and then filtered and centrifuged under conditions which make it possible to obtain a product containing the cells from which the DNA can be extracted.

6. Primer having a sequence chosen from the SEQ ID 1 to 12 sequences.

7. Kit for detection by amplification of DNA and intended for carrying out the process according to one of Claims 3 to 5, characterized in that it comprises the primers according to Claim 6.
